# EUROPEAN PATENT APPLICATION

(11) **EP 4 170 675 A1**
(43) Date of publication of application: **26.04.2023**
(21) Application number: 22202266.7
(22) Date of filing: 18.10.2022
(51) Int. Cl.: G16H 40/40, G16H 40/63, G16H 50/20

(54) **AUTOMATIC POSITIONING AND FORCE ADJUSTMENT IN ENDOSCOPY**

(30) Priority: 20.10.2021 US 202163262794 P; 08.11.2021 US 202163263732 P; 22.04.2022 US 202263363446 P
(71) Applicant: Olympus Corporation, Hachioji-shi Tokyo 192-8507 (JP)
(72) Inventor: Mino, Hiroyuki, Brooklyn Park, 55455 (US); Horio, Hirokazu, Brooklyn Park, 55455 (US); Sakaguchi, Seiichiro, Brooklyn Park, 55455 (US); Ishikake, Makoto, Brooklyn Park, 55455 (US)
(74) Representative: Gunzelmann, Rainer

(57) **Abstract**

Systems, devices, and methods for robotically maneuvering a steerable elongate instrument in a target anatomy and controlling imaging scan of the target anatomy are disclosed. A endoscopic system comprises a steerable elongate instrument to be robotically positioned and navigated in a target anatomy, and a controller to receive patient information including an endoscopic image of the target anatomy, apply the endoscopic image to at least one trained machine-learning (ML) model to recognize the target anatomy, and estimate values of one or more contrast and scan parameters for contrast medium administration and imaging scan. The controller circuit can provide the estimated one or more contrast and scan parameters to a user or a process. In an example, the controller can robotically facilitate injection of contrast medium and imaging of the target anatomy in accordance with the estimated contrast and scan parameters.

## Description

### CROSS REFERENCE TO RELATED APPLICATIONS

This application is based upon and claims the benefit of priority from U.S. Provisional Patent Application Serial No. 63/262,794, filed on October 20, 2021, U.S. Provisional Patent Application Serial No. 63/263,732, filed on November 8, 2021, and U.S. Provisional Patent Application Serial No. 63/363,446, filed on April 22, 2022, the entire contents of which are incorporated herein by reference.

### FIELD OF THE DISCLOSURE

The present document relates generally to endoscopic systems, and more particularly to robotic endoscopic systems and techniques for automatically adjusting cannulation or endoscope navigation and imaging scan in an endoscopic procedure.

### BACKGROUND

Endoscopes have been used in a variety of clinical procedures, including, for example, illuminating, imaging, detecting and diagnosing one or more disease states, providing fluid delivery (e.g., saline or other preparations via a fluid channel) toward an anatomical region, providing passage (e.g., via a working channel) of one or more therapeutic devices or biological matter collection devices for sampling or treating an anatomical region, and providing suction passageways for collecting fluids (e.g., saline or other preparations), among other procedures.

Examples of such anatomical region can include gastrointestinal tract (e.g., esophagus, stomach, duodenum, pancreaticobiliary duct, intestines, colon, and the like), renal area (e.g., kidney(s), ureter, bladder, urethra) and other internal organs (e.g., reproductive systems, sinus cavities, submucosal regions, respiratory tract), and the like.

In conventional endoscopy, the distal portion of the endoscope can be configured for supporting and orienting a therapeutic device, such as with the use of an elevator. In some systems, two endoscopes can work together with a first endoscope guiding a second endoscope inserted therein with the aid of the elevator. Such systems can be helpful in guiding endoscopes to anatomic locations within the body that are difficult to reach. For example, some anatomic locations can only be accessed with an endoscope after insertion through a circuitous path.

Peroral cholangioscopy is a technique that permits direct endoscopic visualization, diagnosis, and treatment of various disorders of patient biliary and pancreatic ductal system using miniature endoscopes and catheters inserted through the accessory port of a duodenoscope. Peroral cholangioscopy can be performed by using a dedicated cholangioscope that is advanced through the accessory channel of a duodenoscope, as used in Endoscopic Retrograde Cholangio-Pancreatography (ERCP) procedures. ERCP is a technique that combines the use of endoscopy and fluoroscopy to diagnose and treat certain problems of the biliary or pancreatic ductal systems, including the liver, gallbladder, bile ducts, pancreas, or pancreatic duct. In ERCP, an cholangioscope (also referred to as an auxiliary scope, or a "daughter" scope) can be attached to and advanced through a working channel of a duodenoscope(also referred to as a main scope, or a "mother" scope). Typically, two separate endoscopists operate each of the "mother-daughter" scopes. Although biliary cannulation can be achieved directly with the tip of the cholangioscope, most endoscopists prefer cannulation over a guidewire. A tissue retrieval device can be inserted through the cholangioscope to retrieve biological matter (e.g., gallstones, bill duct stones, cancerous tissue) or to manage stricture or blockage in bile duct.

Peroral cholangioscopy can also be performed by inserting a small- diameter dedicated endoscope directly into the bile duct, such as in a Direct Per-Oral Cholangioscopy (DPOC) procedure. In DPOC, a slim endoscope (cholangioscope) can be inserted into patient mouth, pass through the upper GI tract, and enter into the common bile duct for visualization, diagnosis, and treatment of disorders of the biliary and pancreatic ductal systems.

Precise positioning and navigation of a steerable instrument (e.g., an endoscope, a catheter, or a cannula) is important for a successful endoscopic procedure like ERCP and DPOC. Conventionally, operating physicians manually position and navigate the endoscope or other instruments based on endoscopic images and fluoroscopy images and their experiences. In some cases this can be challenging for inexperience physicians, especially if the patient undergoing the procedure has surgically altered or otherwise difficult anatomy.

### SUMMARY

The present disclosure recognizes several technological problems to be solved with conventional endoscopes, such as duodenoscopes used for diagnostics and retrieval of sample biological matter. One of such problems is increased difficulty in navigating endoscopes, and instruments inserted therein, to locations in anatomical regions deep within a patient. For example, in ERCP procedures, as the duodenoscope, the cholangioscope, and the tissue retrieval device become progressively smaller due to being inserted sequentially in progressively smaller lumens, it has become more difficult to maneuver and navigate the endoscope through the patient anatomy, maintain endoscope stabilization, and maintain correct cannulation position in a narrow space (e.g., the bile duct). It can also be difficult to maintain an appropriate cannulation angle due to limited degree of freedom in scope elevator. Cannulation and endoscope navigation require advanced surgical skills and manual dexterity, which can be particularly challenging for less-experienced operating physicians (e.g., surgeons or endoscopists).

Another challenge in conventional endoscopy is a high degree of variability of patient anatomy, especially patients with surgically altered or otherwise difficult anatomy. For example, in ERCP procedures, some patients may have altered anatomy to a portion of the GI tract or the pancreaticobiliary system (e.g., the ampulla). In some patients, stricture ahead of pancreas can compress the stomach and part of duodenum, making it difficult to navigate the duodenoscope in a limited lumen of the compressed duodenum and to navigate the cholangioscope to reach the duodenal papilla, the point where the dilated junction of the pancreatic duct and the bile duct (ampulla of Vater) enter the duodenum. In another example, some patients have alternated papilla anatomy. With the duodenoscope designed to be stable in the duodenum, it can be more difficult to reach the duodenal papilla in surgically altered anatomy. Conventional endoscopic systems generally lack the capability of providing cannulation and endoscope navigation guidance based on patient's unique anatomy.

Conventional endoscopic systems generally lack the capability of automatic endoscope positioning and force and motion adjustment during a procedure. The operating physician manually positions and navigates the endoscope based on real-time endoscopic images and fluoroscopy images and their experience. This generally requires extensive training and experience over years, and can be challenging for inexperience physicians, especially in patients with difficult or surgically altered anatomy, as discussed above. Manual endoscope maneuvering can also be labor-intensive as the operating physician (e.g., a surgeon or endoscopist) usually has to hold the endoscope during the entirety of the procedure. Hand tremor may reduce precision of endoscope positioning and navigation, and cause unstable endoscopic view. This may reduce procedure accuracy, efficiency, and success rate.

Some endoscopic procedures such as ERCP inherently require fluoroscopy and characteristically include both standard radiography and cineradiography, which utilizes rapidly changing levels of radiation exposure. Although a patient receiving a small amount of radiation may experience no immediate signs or symptoms, excess radiation exposure during the procedures is of great concern due to its potential carcinogenic effects. In present image-guided endoscopic procedures such as ERCP, fluoroscopy time is often managed by the operators according to their preferences. There is an unmet need for automatic and effective reduction of radiation exposure during procedures such as ERCP.

Fluoroscopy is typically accompanied by radiopaque contrast medium administration to enhance visualization of organs or structures being studied and to help minimize radiation exposure to patient. In some patients, contrast media administration may pose a risk of adverse effects such as contrast- induced acute kidney injury. To reduce the incidence of nephrotoxicity, the amount of contrast medium utilized should be minimized as much as possible and not exceed the calculated maximum allowable contrast dose. Clinically, hydration with normal saline is a suitable strategy for most patients at risk of nephrotoxicity. On the other hand, for procedures such as ERCP, injecting contrast medium into the bile duct can be difficult due to the limited or restricted anatomical site. Automatic contrast medium administration, including determining a proper amount (volume), flow rate, injection duration, and injection site of contrast medium can help reduce the risk of contrast-induced adverse effects while ensuring adequate visualization under fluoroscopy.

The present disclosure can help solve these and other problems by providing systems, devices and method for cannulation and endoscope navigation and automatic imaging scan control in a robotically assisted endoscopic procedure. The automatic cannulation and endoscope navigation can be achieved using an artificial intelligence (AI) platform. According to one example, an endoscopic system comprises a steerable elongate instrument configured to be robotically positioned and navigated in a target anatomy of a patient via an actuator such as included in a robot arm. The system comprises a controller to receive patient information including an image of the target anatomy, apply the target anatomy image (optionally together with other patient and device information) to at least one trained machine-learning (ML) model to recognize the target anatomy and to estimate values (e.g., forces, angles, etc.) for one or more cannulation or navigation parameters. The controller can provide a control signal to the actuator to robotically facilitate operation of the steerable elongate instrument (e.g., positioning or navigation) in the target anatomy in accordance with the estimated values of the cannulation or navigation parameters. In addition, operation of the actuator may be adjusted in real time so as to navigate the steerable elongate instrument based on the information (e.g., imaging data) of the patient's anatomy acquired during the procedure.

According to another example, an endoscopic system comprises a steerable elongate instrument configured to be positioned and navigated in a target anatomy of a patient, and a controller circuit. The controller circuit can be configured to receive patient information including an image of the target anatomy, apply the image of the target anatomy to at least one trained machine-learning (ML) model to recognize the target anatomy and to estimate one or more contrast medium parameters (e.g., a volume, a flow rate, or an injection duration of the contrast medium), or one or more scan parameters (e.g., scan timing, scan duration, scan direction, or a scan delay with respect to the administration of the contrast medium). The controller circuit can provide the estimated one or more contrast and scan parameters to a user or a process. In an example, the controller can provide a control signal to an actuator to robotically facilitate administration of contrast medium via the steerable elongate instrument or imaging of the target anatomy in accordance with the estimated one or more contrast and scan parameters.

The AI-based automatic positioning and navigation system described in this disclosure can improve target anatomy recognition (e.g., duodenal papilla and duct system) and endoscope navigation accuracy and efficiency (e.g., such as navigating into duodenal papilla, through tight strictures, or the hilum of the liver), and help maintain correct cannula position and endoscope stabilization. Enhanced automation in endoscopic procedure can help ease physicians' burden of manual surgical planning, reduce variability of procedure outcome due to variations in experience and dexterity across operating physicians (e.g., surgeons or endoscopists), and improve the endoscopic procedure prognostic predictability. The AI-based determination or adjustment of contrast and scan parameters can help reduce radiation exposure during an image-guided endoscopic procedure such as ERCP. Compared to manual procedures, the robotics and the AI-based positioning and navigation as described in this document can alleviate physician fatigue and prevent hand trembling, and improve procedure efficiency, accuracy, patient safety, and overall endoscopic procedure success rate.

Example 1 is an endoscopic system, comprising: a steerable elongate instrument configured to be positioned and navigated in a target anatomy of a patient; and a controller circuit configured to: receive patient information including an image of the target anatomy; apply the image of the target anatomy to at least one trained machine-learning (ML) model to recognize the target anatomy and to estimate one or more contrast and scan parameters for an imaging scan of the target anatomy; and provide the estimated one or more contrast and scan parameters to a user or a process.

In Example 2, the subject matter of Example 1 optionally includes the received image of the target anatomy that can include an endoscopic image.

In Example 3, the subject matter of any one or more of Examples 1-2 optionally includes the at least one trained ML model that can include a first trained ML model and a second trained ML model different from the first trained ML model, and the controller circuit that can be configured to apply the image of the target anatomy to the first trained ML model to recognize the target anatomy, and to apply the image of the target anatomy to the second trained ML model to estimate the one or more contrast and scan parameters for the imaging scan of the target anatomy.

In Example 4, the subject matter of any one or more of Examples 1-3 optionally includes the controller circuit that can be further configured to provide a control signal to an actuator to robotically facilitate (i) administration of contrast medium via the steerable elongate instrument or (ii) imaging of the target anatomy in accordance with the estimated one or more contrast and scan parameters.

In Example 5, the subject matter of Example 4 optionally includes the steerable elongate instrument that can include an endoscope or a steerable injection device, the estimated one or more contrast and scan parameters that can include a contrast medium parameter, the controller circuit that can be configured to provide the control signal to the actuator to robotically facilitate operation of the steerable elongate instrument to administer the contrast medium in accordance with the contrast medium parameter.

In Example 6, the subject matter of Example 5 optionally includes the contrast medium parameter that can include one or more of a volume, a flow rate, or an injection duration of the contrast medium.

In Example 7, the subject matter of any one or more of Examples 5-6 optionally includes the target anatomy that can include a portion of a pancreaticobiliary system of the patient, and the contrast medium parameter that can include a contrast medium injection site of the pancreaticobiliary system.

In Example 8, the subject matter of any one or more of Examples 4-7 optionally includes the estimated one or more contrast and scan parameters that can include a scan parameter for imaging the target anatomy; and the controller circuit that can be configured to provide the control signal to the actuator to robotically facilitate operation of an imaging device to take an imaging scan of the target anatomy in accordance with the scan parameter.

In Example 9, the subject matter of Example 8 optionally includes the scan parameter that can include one or more of a scan timing, scan duration, scan direction, or a scan delay with respect to the administration of the contrast medium.

In Example 10, the subject matter of any one or more of Examples 1- 9 optionally includes the target anatomy that can include a portion of a pancreaticobiliary system of the patient, and the controller circuit that can be configured to apply the image of the target anatomy to the trained ML model to recognize stenosis in the pancreaticobiliary system.

In Example 11, the subject matter of any one or more of Examples 1- 10 optionally includes a training module configured to train the ML model using a training dataset comprising procedure data from past endoscopic procedures on a plurality of patients, the procedure data comprising (i) endoscopic images of target anatomies of the plurality of patients, and (ii) assessments of respective contrast and scan parameters used to produce the endoscopic images.

In Example 12, the subject matter of Example 11 optionally includes the training module that can be configured to train the ML model using supervised learning or deep learning.

In Example 13, the subject matter of any one or more of Examples 1- 12 optionally includes the controller circuit that can be configured to estimate the one or more contrast and scan parameters further using viscosity information of a contrast medium.

In Example 14, the subject matter of any one or more of Examples 1- 13 optionally includes an output unit configured to present the estimated one or more contrast and scan parameters to a user to assist the user in administering a contrast medium or imaging the target anatomy.

Example 15 is a method of robotically maneuvering an steerable elongate instrument in a target anatomy and controlling imaging scan of the target anatomy via an endoscopic system, the method comprising: providing patient information including an image of the target anatomy; applying the target anatomy image to at least one trained machine-learning (ML) model and recognizing the target anatomy and estimating one or more contrast and scan parameters for an imaging scan of the target anatomy; and providing the estimated one or more contrast and scan parameters to a user or a process.

In Example 16, the subject matter of Example 15 optionally includes applying the target anatomy image to at least one trained ML model that can include: applying the image of the target anatomy to a first trained ML model to recognize the target anatomy; and applying the image of the target anatomy to a second trained ML model to estimate the one or more contrast and scan parameters for the imaging scan of the target anatomy, the second trained ML model different from the first trained ML model.

In Example 17, the subject matter of any one or more of Examples 15-16 optionally includes providing a control signal to an actuator to robotically facilitate (i) administration of contrast medium via the steerable elongate instrument or (ii) imaging of the target anatomy in accordance with the estimated one or more contrast and scan parameters.

In Example 18, the subject matter of Example 17 optionally includes the estimated one or more contrast and scan parameters that can include a contrast medium parameter for administering a contrast medium; and the control signal that can be provided to the actuator to robotically facilitate operation of the steerable elongate instrument to administer the contrast medium is in accordance with the contrast medium parameter.

In Example 19, the subject matter of Example 18 optionally includes the contrast medium parameter that can include one or more of a volume, a flow rate, or an injection duration of the contrast medium.

In Example 20, the subject matter of any one or more of Examples 18-19 optionally includes the target anatomy that can include a portion of a pancreaticobiliary system of the patient, and the contrast medium parameter that can include a contrast medium injection site of the pancreaticobiliary system.

In Example 21, the subject matter of any one or more of Examples 17-20 optionally includes the estimated one or more contrast and scan parameters that can include a scan parameter for imaging the target anatomy; and the control signal that can be provided to the actuator to robotically facilitate operation of an imaging device to take an imaging scan of the target anatomy in accordance with the scan parameter.

In Example 22, the subject matter of Example 21 optionally includes the scan parameter that can include one or more of a scan timing, scan duration, scan direction, or a scan delay with respect to the administration of the contrast medium.

In Example 23, the subject matter of any one or more of Examples 15-22 optionally includes training the ML model, via a training module of the endoscopic system, using a training dataset comprising stored endoscopic procedure data of a plurality of patients, the stored data comprising (i) endoscopic images of target anatomies of the plurality of patients, and (ii) assessments of respective contrast and scan parameters used to produce the endoscopic images.

In Example 24, the subject matter of any one or more of Examples 15-23 optionally includes presenting the estimated one or more contrast and scan parameters to a user via an output unit to assist the user in administering a contrast medium or imaging the target anatomy.

The presented techniques are described in terms of health-related procedures, but are not so limited. This summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims and their legal equivalents.

### BRIEF DESCRIPTION OF THE DRAWING

FIGS. 1-2 are schematic diagrams illustrating an example of an endoscopy system for use in endoscopic procedures such as an ERCP procedure.
FIGS. 3A-3B are diagrams illustrating an example of peroral cholangioscopy involving direct insertion of a cholangioscope into patient bile duct as in a DPOC procedure, and a portion of patient anatomy where the procedure is performed.
FIG. 4 is a diagram illustrating an example of mother-daughter endoscopes used in an ERCP procedure, and a portion of patient anatomy where the procedure is performed.
FIG. 5 illustrates generally an example of a robotic endoscopic surgical system and at least a portion of the environment in which the system operates.
FIG. 6 is a block diagram illustrating an example of a robotically assisted cannulation and navigation system.
FIGS. 7A-7B are diagrams illustrating an example of training a ML model for papilla recognition, and using the trained ML model to detect and localize a papilla from a live endoscopic image.
FIGS. 7C-7D are diagrams illustrating an example of training a ML model for estimating one or more contrast and scan parameters for an imaging scan of a target anatomy.
FIG. 8 is a flow chart illustrating an example method for robotically positioning and navigating a steerable elongate instrument in a target anatomy via an endoscopic system.
FIG. 9 is a block diagram illustrating an example machine upon which any one or more of the techniques (e.g., methodologies) discussed herein may perform.

### DETAILED DESCRIPTION

This document describes systems, devices, and methods for automatic cannulation and endoscope navigation in a robotically assisted endoscopic procedure using artificial intelligence (AI) technology. According to one embodiment, an endoscopic system comprises a steerable elongate instrument configured to be robotically positioned and navigated in a target anatomy of a patient via an actuator such as included in a robot arm. The system comprises a controller to receive patient information including an image of the target anatomy, apply the target anatomy image (optionally together with other patient and device information) to at least one trained machine-learning (ML) model to recognize the target anatomy and to estimate values (e.g., forces, angles, etc.) for one or more cannulation or navigation parameters. The controller can provide a control signal to the actuator to robotically facilitate operation of the steerable elongate instrument (e.g., positioning and navigation) in the target anatomy in accordance with the estimated values of the cannulation or navigation parameters. In some examples, operation of the actuator may be adjusted in real time so as to navigate the steerable elongate instrument based on the information (e.g., imaging data) of the patient's anatomy acquired during the procedure.

Although the discussion of robotically assisted and automatic cannulation or navigation in the present document is focused on endoscopes such as those used in ERCP or DPOC procedures, the systems, apparatus, and methods described herein, particularly the automatic cannulation and endoscope navigation adjustment, can be used in a wide variety of robotically assisted procedures involving a use of steerable endoluminal instrument beyond diagnostic or therapeutic endoscopes, which may include, for example, cannulas, catheters, guidewires, or guide sheaths, among others.

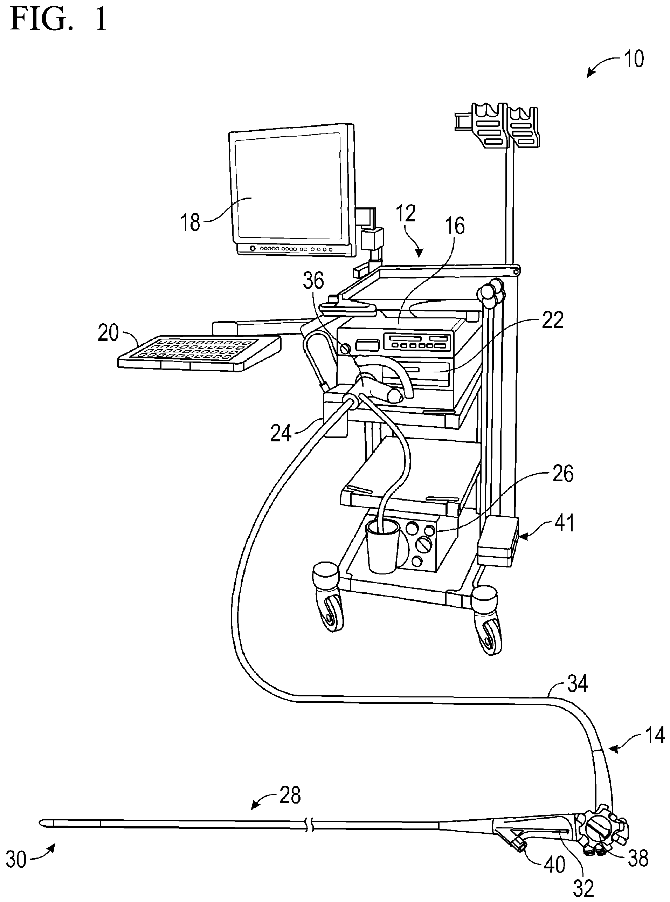

FIG. 1 is a schematic diagram illustrating an example of an endoscopy system 10 for use in endoscopic procedures, such as an ERCP procedure. The system 10 comprises an imaging and control system 12 and an endoscope 14.

The endoscopy system 10 is an illustrative example of an endoscopy system suitable for patient diagnosis and/or treatment using the systems, devices and methods described herein, such as tethered and optically enhanced biological matter and tissue collection, retrieval and storage devices and biopsy instruments that can be used for obtaining samples of tissue or other biological matter to be removed from a patient for analysis or treatment of the patient. According to some examples, the endoscope 14 can be insertable into an anatomical region for imaging and/or to provide passage of or attachment to (e.g., via tethering) one or more sampling devices for biopsies, or one or more therapeutic devices for treatment of a disease state associated with the anatomical region.

The imaging and control system 12 can comprise a control unit 16, an output unit 18, an input unit 20, a light source 22, a fluid source 24, and a suction pump 26. The imaging and control system 12 can include various ports for coupling with endoscopy system 10. For example, the control unit 16 can include a data input/output port for receiving data from and communicating data to the endoscope 14. The light source 22 can include an output port for transmitting light to the endoscope 14, such as via a fiber optic link. The fluid source 24 can comprise one or more sources of air, saline or other fluids, as well as associated fluid pathways (e.g., air channels, irrigation channels, suction channels) and connectors (barb fittings, fluid seals, valves and the like). The fluid source 24 can be in communication with the control unit 16, and can transmit one or more sources of air or fluids to the endoscope 14 via a port. The fluid source 24 can comprise a pump and a tank of fluid or can be connected to an external tank, vessel or storage unit. The suction pump 26 can comprise a port used to draw a vacuum from the endoscope 14 to generate suction, such as for withdrawing fluid from the anatomical region into which the endoscope 14 is inserted.

The output unit 18 and the input unit 20 can be used by a human operator and/or a robotic operator of endoscopy system 10 to control functions of endoscopy system 10 and view output of endoscope 14. In some examples, the control unit 16 can additionally be used to generate signals or other outputs for treating the anatomical region into which the endoscope 14 is inserted. Examples of such signals or outputs can include electrical output, acoustic output, a radio- frequency energy output, a fluid output and the like for treating the anatomical region with, for example, cauterizing, cutting, freezing and the like.

The endoscope 14 can interface with and connect to the imaging and control system 12 via a coupler section 36. In the illustrated example, the endoscope 14 comprises a duodenoscope that may be use in an ERCP procedure, though other types of endoscopes can be used with the features and teachings of the present disclosure. The endoscope 14 can comprise an insertion section 28, a functional section 30, and a handle section 32, which can be coupled to a cable section 34 and the coupler section 36.

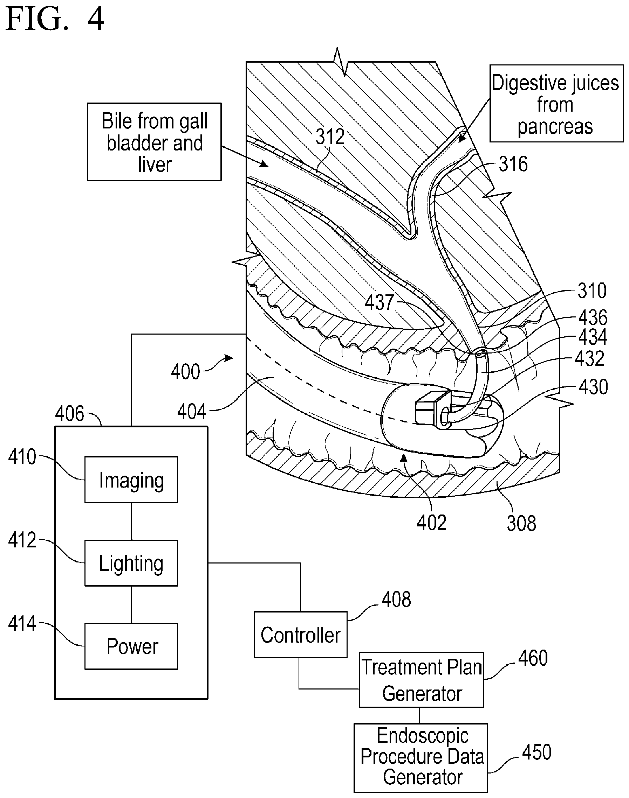

The insertion section 28 can extend distally from the handle section 32, and the cable section 34 can extend proximally from the handle section 32. The insertion section 28 can be elongate and include a bending section, and a distal end to which functional section 30 can be attached. The bending section can be controllable (e.g., by control knob 38 on the handle section 32) to maneuver the distal end through tortuous anatomical passageways (e.g., stomach, duodenum, kidney, ureter, etc.). Insertion section 28 can also include one or more working channels (e.g., an internal lumen) that can be elongate and support insertion of one or more therapeutic tools of functional section 30, such as a cholangioscope as shown in FIG. 4. The working channel can extend between handle section 32 and functional section 30. Additional functionalities, such as fluid passages, guidewires, and pull wires can also be provided by insertion section 28 (e.g., via suction or irrigation passageways, and the like).

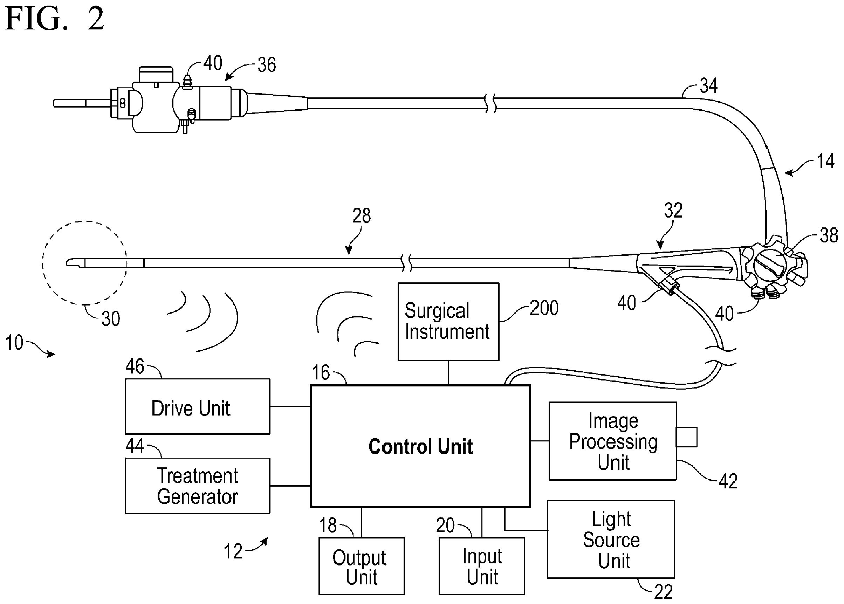

The handle section 32 can comprise a control knob 38 and ports 40. The ports 40 can be configured to couple various electrical cables, guidewires, auxiliary scopes, tissue collection devices of the present disclosure, fluid tubes and the like to handle section 32 for coupling with insertion section 28. The control knob 38 can be coupled to a pull wire, or other actuation mechanisms, extending through insertion section 28. The control knob 38 can be used by a user to manually advance or retreat the insertion section 28 of the endoscope 14, and to adjust bending of a bending section at the distal end of the insertion section 28. In some examples, an optional drive unit 46 (FIG. 2) can be used to provide motorized drive for advancing a distal section of endoscope 14 under the control of the control unit 16.

The imaging and control system 12, according to examples, can be provided on a mobile platform (e.g., cart 41) with shelves for housing light source 22, suction pump 26, image processing unit 42 (FIG. 2), etc. Alternatively, several components of the imaging and control system 12 shown in FIGS. 1 and 2 can be provided directly on the endoscope 14 such that the endoscope is "self-contained."

The functional section 30 can comprise components for treating and diagnosing anatomy of a patient. The functional section 30 can comprise an imaging device, an illumination device, and an elevator. The functional section 30 can further comprise optically enhanced biological matter and tissue collection and retrieval devices. For example, the functional section 30 can comprise one or more electrodes conductively connected to handle section 32 and functionally connected to the imaging and control system 12 to analyze biological matter in contact with the electrodes based on comparative biological data stored in the imaging and control system 12. In other examples, the functional section 30 can directly incorporate tissue collectors.

In some examples, the endoscope 14 can be robotically controlled, such as by a robot arm attached thereto. The robot arm can automatically, or semi- automatically (e.g., with certain user manual control or commands) position and navigate the elongate portion (including the functional section 30 and/or the insertion section 28) of the endoscope 14 in the target anatomy via an actuator. In accordance with various examples discussed in this document, a controller can use the artificial intelligence (AI) technology to determine cannulation and endoscope navigation parameters for use in a robotically assisted endoscopic procedure, and generate a control signal to the actuator of the robot arm to facilitate operation (e.g., to adjust the position, angle, force, and/or navigation) of the elongate portion of the endoscope in the target anatomy in accordance with the determined cannulation or navigation parameters.

FIG. 2 is a schematic diagram of the endoscopy system 10 shown in FIG. 1, which comprises the imaging and control system 12 and the endoscope 14. FIG. 2 schematically illustrates components of the imaging and control system 12 coupled to the endoscope 14, which in the illustrated example comprises a duodenoscope. The imaging and control system 12 can comprise a control unit 16, which can include or be coupled to an image processing unit 42, a treatment generator 44, and a drive unit 46, as well as the light source 22, the input unit 20, and the output unit 18 as discussed above with reference to FIG. 1. The control unit 16 can comprise, or can be in communication with, a surgical instrument 200 comprising a device configured to engage tissue and collect and store a portion of that tissue and through which an imaging device (e.g., a camera) can view target tissue via inclusion of optically enhanced materials and components. The control unit 16 can be configured to activate an imaging device (e.g., a camera) at the functional section of the endoscope 14 to view target tissue distal of surgical instrument 200 and endoscopy system 10, which can be fabricated of a translucent material to minimize the impacts of the camera being obstructed or partially obstructed by the tissue retrieval device. Likewise, the control unit 16 can be configured to activate the light source 22 to shine light on the surgical instrument 200, which can include select components that are configured to reflect light in a particular manner, such as tissue cutters being enhanced with reflective particles.

The image processing unit 42 and the light source 22 can each interface with the endoscope 14 (e.g., at the functional section 30) by wired or wireless electrical connections. The imaging and control system 12 can accordingly illuminate an anatomical region using the light source 22, collect signals representing the anatomical region, process signals representing the anatomical region using the image processing unit 42, and display images representing the anatomical region on the output unit 18. The imaging and control system 12 can include the light source 22 to illuminate the anatomical region using light of desired spectrum (e.g., broadband white light, narrowband imaging using preferred electromagnetic wavelengths, and the like). The imaging and control system 12 can connect (e.g., via an endoscope connector) to the endoscope 14 for signal transmission (e.g., light output from light source, video signals from the imaging device such as positioned at the distal portion of the endoscope 14, diagnostic and sensor signals from a diagnostic device, and the like).

The treatment generator 44 can generate a treatment plan, which can be used by the control unit 16 to control the operation of the endoscope 14, or to provide with the operating physician a guidance for maneuvering the endoscope 14, during an endoscopic procedure. In an example, the treatment generator 44 can generate an endoscope navigation plan, including estimated values for one or more cannulation or navigation parameters (e.g., an angle, a force, etc.) for maneuvering the steerable elongate instrument, using patient information including an image of the target anatomy. The endoscope navigation plan can help guide the operating physician to cannulate and navigate the endoscope in the patient anatomy. The endoscope navigation plan may additionally or alternatively be used to robotically adjust the position, angle, force, and/or navigation of the endoscope or other instrument. Examples of AI-based target anatomy recognition and cannulation or navigation parameter estimation, and robotic positioning and navigation in an endoscopic procedure are discussed below with reference to FIGS. 4-6.

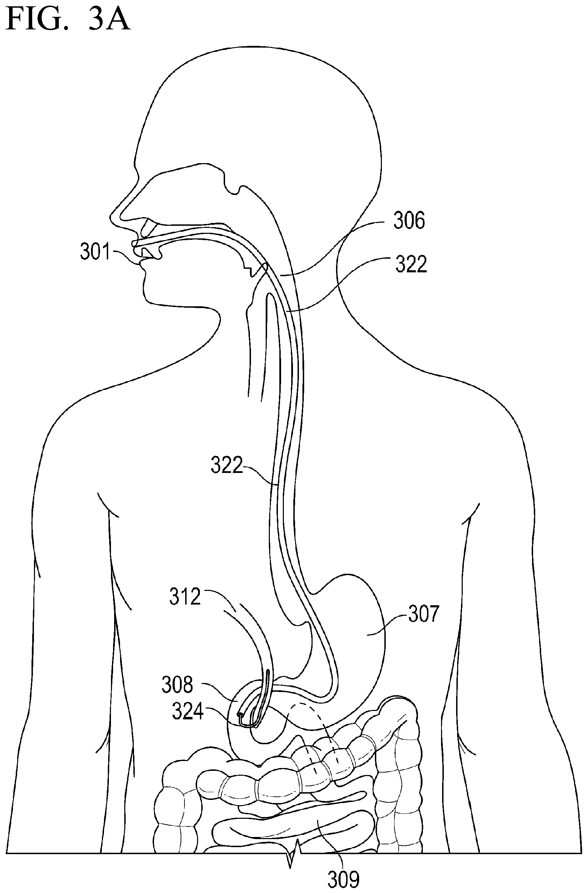

FIGS. 3A-3B are diagrams illustrating an example of peroral cholangioscopy performed via direct insertion of a cholangioscope 324 into the bile duct, as in a DPOC procedure, and a portion of patient anatomy where the procedure is performed. The cholangioscope 324 is nested inside of a guide sheath 322, and inserted perorally into a patient to reach duodenum 308. Duodenum 308 comprises an upper part of the small intestine. The guide sheath 322 can extend into mouth 301, through esophagus 306, through stomach 307 to reach the duodenum 308.

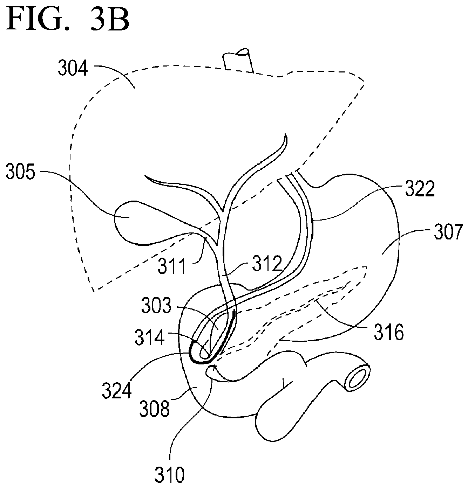

Before reaching intestines 309, the guide sheath 322 can position the cholangioscope 324 proximate common bile duct 312. The common bile duct 312 carries bile from the gallbladder 305 and liver 304, and empties the bile into the duodenum 308 through sphincter of Oddi 310 (FIG. 3B). The cholangioscope 324 can extend from guide sheath 322 to extend into common bile duct 312. In some examples, steering features of guide sheath 322 (e.g., pull wire) can be used to facilitate navigating and bending of cholangioscope 324 through stomach 307, in addition to direct steering of cholangioscope 324 via the pull wires. For example, navigation of the Pyloric canal and Pyloric sphincter can be difficult to navigate using only an endoscope. Thus, the guide sheath 322 can be used to turn or bend elongate body of cholangioscope 324, or reduce the amount of steering or bending of the elongate body of the cholangioscope 324 required by pull wires, to facilitate traversing the Pyloric sphincter.

FIG. 3B is a schematic view of duodenum 308 connected to common bile duct 312 via duodenal papilla 314. Common bile duct 312 can branch off into pancreatic duct 316 and gallbladder duct 311. Duodenal papilla 314 can include sphincter of Oddi 310 that controls flow of bile and pancreatic juice into the intestine (duodenum). Pancreatic duct 316 can lead to pancreas 303. Pancreatic duct 316 carries pancreatic juice from pancreas 303 to the common bile duct 312.

Gallbladder duct 311 can lead to gallbladder 305. In some patients, it can be difficult to navigate surgical instruments to duodenal papilla 314. It can also be difficult to navigate a surgical instrument into common bile duct 312 via insertion through duodenal papilla 314. Therefore, it is common during medical procedures to cut sphincter of Oddi 310 to enlarge duodenal papilla 314 to allow for easier access of instrument into common bile duct 312.

FIG. 4 is a diagram illustrating an example of mother-daughter endoscopes used in an ERCP procedure, and a portion of patient anatomy where the procedure is performed. The mother-daughter endoscopes comprise an auxiliary scope 434 (cholangioscope) attached to and advanced through a lumen 432 of a main scope 400 (duodenoscope). The auxiliary scope 434 can comprise a lumen 436. The distal portion of the main scope 400 positioned in patient duodenum 308 comprises a functional module 402, an insertion section module 404, and a control module 406. The control module 406 can include, or be coupled to, a controller 408. Similar to the discussion above with respect to FIG. 1, the control module 406 can include other components, such as those described with reference to endoscopy system 10 (FIG. 1) and control unit 16 (FIG. 2). Additionally, the control module 406 can comprise components for controlling an imaging device (e.g., a camera) and a light source connected to the auxiliary scope 434, such as an imaging unit 410, a lighting unit 412 and a power unit 414. The main scope 400 can be configured similarly as endoscope 14 of FIGS. 1 and 2.

The functional module 402 of the main scope 400 can comprise an elevator portion 430. The auxiliary scope 434 can itself include functional components, such as camera lens 437 and a light lens (not illustrated) coupled to control module 406, to facilitate navigation of the auxiliary scope 434 from the main scope 400 through the anatomy and to facilitate viewing of components extending from lumen 432.

In ERCP, the auxiliary scope 434 can be guided into the sphincter of Oddi 310. Therefrom, a surgeon operating the auxiliary scope 434 can navigate the auxiliary scope 434 through the lumen 432 of the main scope toward the gallbladder 305, liver 304, or other locations in the gastrointestinal system to perform various procedures. In some examples, the auxiliary scope 434 can be used to guide an additional device to the anatomy to obtain biological matter (e.g., tissue), such as by passage through or attachment to lumen 436.

The biological sample matter can be removed from the patient, typically by removal of the additional device from the auxiliary device, so that the removed biological matter can be analyzed to diagnose one or more conditions of the patient. According to several examples, the mother-daughter endoscope assembly (including the main scope 400 and the auxiliary scope 434) can include additional device features, such as forceps or an auger, for gathering and removing cancerous or pre-cancerous matter (e.g., carcinoma, sarcoma, myeloma, leukemia, lymphoma and the like), or performing endometriosis evaluation, biliary ductal biopsies, and the like.

The controller 408 can include, or be coupled to, an endoscopic procedure data generator 450, and a treatment plan generator 460. The endoscopic procedure data generator 450 can generate images of target anatomy, such as duodenal papilla or a portion of the biliary and pancreatic ductal system. In an example, the endoscopic procedure data generator 450 can generate real-time endoscope images of duodenal papilla and its surrounding environment using an imaging sensor on the endoscope, such as a camera located at the functional section 30 of the endoscope 14. In some examples, the endoscopic procedure data generator 450 can acquire other procedure-related information, including sensor information (e.g., sensors associated with the endoscope), device information, patient medical history etc. In some examples, the endoscopic procedure data generator 450 can retrieve, such as from a database, stored control log data (e.g., time-series data) of past endoscopic procedures performed by a plurality of physicians on a plurality of patients. The control log data can represent preferred cannulation and endoscope navigation approaches and habits of physicians with different experience levels.

The treatment plan generator 460, which is an example of the treatment generator 44 as illustrated in FIG. 2, can automatically generate a treatment plan based on the endoscopic images, optionally on other information as well, that are produced by the endoscopic procedure data generator 450. The endoscope navigation plan can include one or more cannulation or navigation parameters (e.g., position, force and/or angle) with respective values. According to various examples, the endoscope navigation plan (e.g., cannulation or navigation parameter values) can be generated or updated using at least one trained machine- learning (ML) model. The cannulation or navigation parameters may be used by a robotic device (e.g., a robot arm) to automatically initiate or adjust position or navigation of an endoscope or other steerable elongate instruments (e.g., a cannula, a catheter, a guidewire, or a guide sheath) in the target anatomy. In some examples, the endoscopic images of the target anatomy, the navigation plan, and the status of positioning and navigation of the steerable elongate instrument may be displayed to the operating physician.

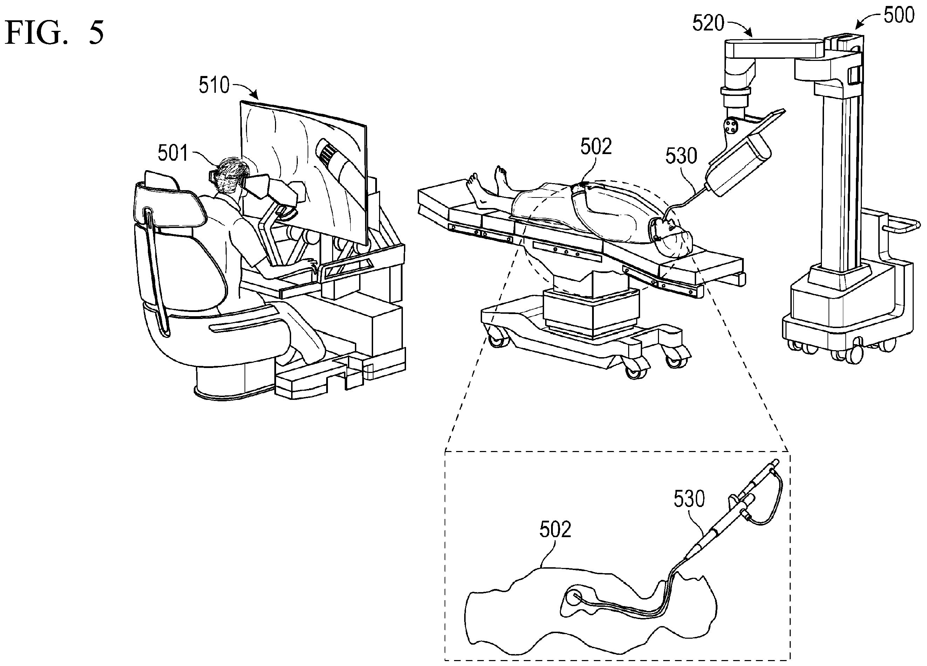

FIG. 5 illustrates generally an example of a robotic endoscopic surgical system 500 and at least a portion of the environment in which the system operates. A surgeon 501 can operate a control console 510 communicatively coupled to a robot arm 520. The robot arm 520 can detachably engage a steerable instrument 530, such as a flexible endoscope as discussed above with reference to FIGS. 3A-3B and 4. The surgeon 501 can perform an endoscopic procedure on patient 502 through the control console 510 that control the motion of the robot arm 520. The robot arm 520 can pass the steerable instrument 530 to a luminal space of the patient 502, and position and navigation of the steerable instrument 530 to a target anatomical location. In some examples, the steerable instrument 530 can include sensors configured to collect patient anatomical information, such as endoscopic images (or videos) of the target anatomy. The collected information can be provided to the surgeon 501, such as being displayed on a monitor integrated into the control console 510. In accordance with various examples discussed herein, the control console 510 may include an artificial intelligence (AI)-based automatic cannulation and endoscope navigation system that can process the received patient information (e.g., endoscopic images, among other sensor information, medical history, etc.), and estimate values for one or more cannulation or navigation parameters (e.g., position, force, angle). The surgeon may use the estimated parameter values as references to adjust the steerable instrument 530 via the robot arm 520, such as adjusting position, direction, or force applied to the steerable instruction 530. Alternatively, the surgeon may command the robot arm 520 to automatically activate or adjust motion of the steerable instrument 530 in accordance with the estimated parameter values. In some examples, the control console 510 can automatically command the robot arm 520 to activate or adjust motion in accordance with the estimated parameter values with little or no human intervention. For example, the surgeon may monitor the procedure and intervene (via manual override) when necessary.

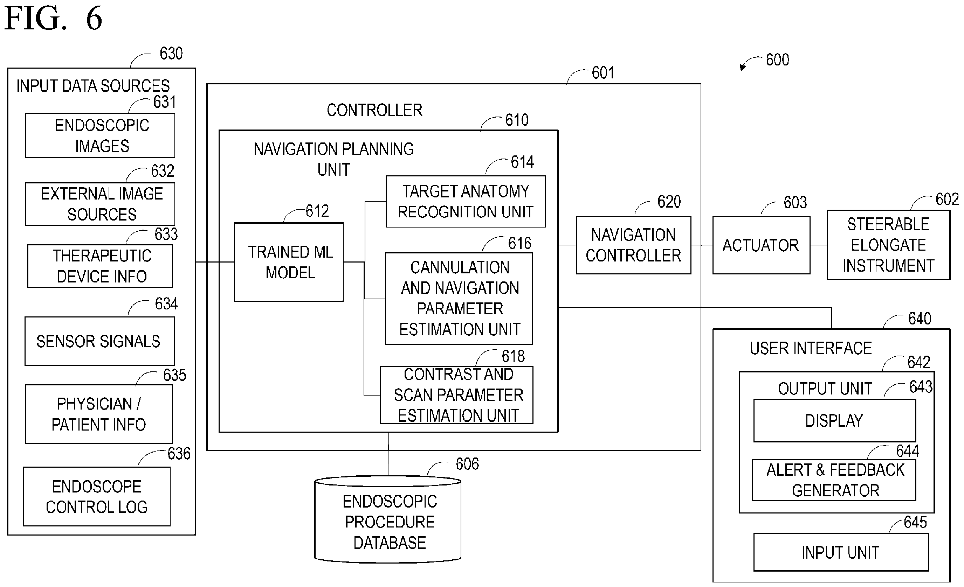

FIG. 6 is a block diagram illustrating by way of example and not limitation a robotically assisted cannulation and navigation system 600. At least portions of the system 600 can be implemented in the control console 510 of the robotic endoscopic surgical system 500. The system 600 can be a part of the control unit 16 in FIG. 1, or the controller 408 in FIG. 4 along with other devices or functional units such as the endoscopic procedure data generator 450 and the treatment plan generator 460.

The robotically assisted navigation system 600 can include a controller 601, an input data source 630, and one or more actuators 603 coupled to a steerable elongate instrument 602 to adjust its position, direction, or force applied thereto during an endoscopic procedure. In some examples, the robotically assisted navigation system 600 can include a user interface device 640.

The controller 601 may include circuit sets comprising one or more other circuits or sub-circuits, including a navigation planning unit 610 and a navigation controller 620. These circuits may, alone or in combination, perform the functions, methods, or techniques described herein. In an example, the controller 601 and the circuits sets therein may be implemented as a part of a microprocessor circuit, which may be a dedicated processor such as a digital signal processor, application specific integrated circuit (ASIC), microprocessor, or other type of processor for processing information including physical activity information.

Alternatively, the microprocessor circuit may be a general-purpose processor that may receive and execute a set of instructions of performing the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

The navigation planning unit 610 may generate an endoscope navigation plan with respect to an anatomical target of interest (e.g., duodenal papilla) using information from one or more input data sources. By way of example and not limitation, the input data sources 630 may include one or more of endoscopic images 631 of the target anatomy, external image sources 632, endo- therapeutic device information 633, sensor signals 634, or physician/patient information 635, as illustrated in FIG. 6. The endoscopic images 631 may include real-time endoscope images of duodenal papilla and its surrounding environment captured by an imaging sensor on the endoscope during an endoscopic procedure, such as the DPOC procedure or an ERCP procedure as described above in reference to FIGS. 3A-3B and FIG. 4, respectively. The external image sources 632 may include images acquired by other imaging devices (other than the endoscope), which may include, for example, X-ray or fluoroscopy images, electrical potential map or an electrical impedance map, CT images, MRI images such as images obtained from Magnetic resonance cholangiopancreatography (MRCP) procedures, or acoustic images such as endoscopic ultrasonography (EUS) images, among others. The endo- therapeutic device information 633 may include specification data, including dimension, shape, and structures of the endoscope used in an ERCP procedure or other steerable instruments such as a cannular, a catheter, or a guidewire. Such device specification information may be used to determine cannulation or navigation parameter values such as the angle and/or the force applied to the device. The sensor signals 634 may be acquired by sensors coupled to the endoscope, or otherwise associated with the patient. In an example, the sensor signals 634 may be acquired by a proximity sensor at a distal portion of the endoscope. Examples of the sensor signals 634 may include position, direction, or proximity of a distal portion of the endoscope relative to duodenal papilla. The physician/patient information 635 may include the operating physician's habits or preference of using the steerable elongate instrument 602, such as a preferred approach for cannulation and endoscope navigation, or past procedures of the similar type to the present procedure performed by the physician and the corresponding procedure outcome (e.g., success/failure, procedure time, prognosis and complications). The physician/patient information 635 may include patient information, such as endoscopic images or other sensor information, patient medical history, etc. The endoscope control log data 636 may include time-series data representing changes in the movement and deflection of the device (e.g., endoscope, catheter, or cannula) on the endoscopic image as being maneuvered during a procedure.

The navigation planning unit 610 may include one or more of a target anatomy recognition unit 614 that can automatically recognize the anatomical target of interest such as from an input image (e.g., endoscopic images 631 and/or external image sources 632), a cannulation and navigation parameter estimation unit 616 that can automatically estimate values for one or more cannulation or navigation parameters, and a contrast and scan parameter estimation unit 618 that can estimate values of one or more contrast medium parameters for controlling administration of a contrast medium, and/or values of one or more scan parameters for taking an imaging scan (e.g., X-rays, fluoroscopy scan, CT scan, MRI scan, among other radiologic imaging exams) of an anatomical target or other regions of interest. The navigation planning unit 610 and the cannulation and navigation parameter estimation unit 616 can use respective trained machine-learning (ML) models to perform respective target recognition and parameter estimation tasks. The ML model 612 may be trained using supervised learning, unsupervised learning, or reinforcement leaning. Examples of ML model architectures and algorithms may include, for example, decision trees, neural networks, support vector machines, or a deep-learning networks, etc. Examples of deep-learning networks include a convolutional neural network (CNN), a recurrent neural network (RNN), a deep belief network (DBN), or a hybrid neural network comprising two or more neural network models of different types or different model configurations.

The ML model 612 may be trained using various sources of information including, for example, one or more of the input data sources 630. In some examples, the ML model 612 may be trained using past procedure data stored in an endoscopic procedure database 606. The past procedure data stored in the database 606 may include procedure data acquired during respective endoscopic procedures performed by a plurality of physicians on a plurality of patients. The endoscopic procedures can be those of the same type as the endoscopic procedure to be performed on the present patient. The stored procedure data can include, for each procedure, endoscopic images or videos showing patient anatomy, cannulation and endoscope navigation routes, progress of cannulation or navigation, among other information. In an example, the stored procedure data can include, for each procedure, one or more cannulation or navigation parameters recorded during the procedure, or obtained by offline analysis the endoscopic images or videos.

To train the ML model, a training dataset can be constructed using one or more of the input data source 630, and past endoscopic procedure data such as selected and retrieved from the endoscopic procedure database 606. In an example, the training data can be screened such that only data of procedures performed by certain physicians (such as those with substantially similar experience levels to the operating physician), and/or data of procedures on certain patients with special requirement (such as those with substantially similar anatomy or patient medical information to the present patient) are included in the training dataset. In an example, the training data can be screened based on a success rate of the procedure, including times of attempts before a successful cannulation or navigation, such that only data of procedures with a desirable success rate achieved within a specified number of attempts are included in the training dataset. In another example, the training data can be screened based on complication associated with the patients. In some examples, particularly in case of a small training dataset (such as due to data screening), the ML model can be trained to generate a treatment plan by extrapolating, interpolating, or bootstrapping the training data, thereby creating a treatment plan specifically tailored to the specific patient and physician. The training of the ML model may be performed continuously or periodically, or in near real time as additional procedure data are made available. The training involves algorithmically adjusting one or more ML model parameters, until the ML model being trained satisfies a specified training convergence criterion.

The cannulation and navigation parameter estimation unit 616 can automatically estimate values for one or more cannulation or navigation parameters, which may include, for example: a position of the distal portion (e.g., the functional section 30 of the endoscope 14 as shown in FIG. 1) of an endoscope or other steerable elongate instrument relative to an anatomical target of interest, such as a distance from the endoscope distal portion to duodenal papilla; a heading direction of the distal portion of the steerable elongate instrument relative to the anatomical target; an insertion angle of a cannula or a surgical element used in cannulation; a protrusion amount of a cannula or a surgical element; a speed or a force applied to the endoscope distal portion or a surgical element; a rotational direction or a cutting area of a surgical element; among others. In some examples, the navigation plan may include a projected navigation path toward the anatomical target of interest.

The navigation controller 620 can generate a control signal to the one or more actuators 603, such as a motor actuating a robot arm. The one or more actuators 603 can be coupled to the steerable elongate instrument 602, such as a proximal portion thereof. Examples of the steerable endoluminal instrument 602 can include diagnostic or therapeutic endoscopes, cannulas, catheters, guidewires, or guide sheaths, among others. In response to the control signal, the one or more actuators 603 can robotically adjust position or navigation of the steerable elongate instrument 602 in the target anatomy in accordance with the one or more cannulation or navigation parameters estimated by the cannulation and navigation parameter estimation unit 616. As some of the canulation or navigation parameters (e.g., positions, angle, direction, navigation path) associated with a cannula or GW are determined based on images (e.g., endoscopic images or other images) generated an imaging system, such canulation or navigation parameters are with reference to the coordinates of the imaging system. To facilitate robotic control of the cannula or GW in accordance with the canulation or navigation parameters, in some examples the coordinates of the robotic system may be registered with the coordinates of the imaging system, such that an anatomical position in the coordinates of the imaging system can be mapped to a corresponding position in the coordinates of the robotic system. Such registration may be performed, for example, by using distinct landmarks whose positions are known in respective coordinate systems. Th registration may be intensity- or feature-based, and can be represented by transformation model (a linear or a non-linear model) that maps the coordinates of imaging system to the coordinates of the robotic system.

In an example, the cannulation and navigation parameter estimation unit 616 can determine a force applied to a cannula or GW (an example of the steerable elongate instrument 602) based on a distance from the distal tip of the cannula/GW to duodenal papilla. Such distance can be determined using sensor signals 634, such as via a proximity sensor at the tip of the cannula/GW. In another example, the distance can be measured by a displacement sensor, disposed on the actuator 603 or a robot arm coupling to the cannula/GW, that measures a length of insertion into duodenal papilla. Additionally or alternatively, the distance can be estimated from the endoscopic images 631. With the information about the distance between the distal tip of the cannula/GW to duodenal papilla, the cannulation and navigation parameter estimation unit 616 can determine a lower force applied to a cannula/GW as the distal tip of the cannula/GW gets closer to duodenal papilla. In an example, the applied force can be inversely proportional to said distance. After the distal tip of the cannula/GW being inserted into and passing through duodenal papilla, the navigation parameter estimation unit 616 can determine a lower level or range of force than the force level or range before the insertion. The navigation controller 620 can then control the actuator 603 (or the robot arm) to apply the distance-dependent force as determined above to the cannula/GW. Dynamically adjusting the force based on the distance to the critical anatomy (e.g., duodenal papilla, common bile duct, and pancreas) as described herein can avoid or reduce damage to pancreatic parenchyma caused by cannula/GW. Robotic assistance can increase the precision of cannula/GW positioning and advancement at or near the critical anatomy, and further reduce the risk of tissue damage due to improper cannulation.

In an example, the cannulation and navigation parameter estimation unit 616 can determine an insertion angle of a cannula or GW (an example of the steerable elongate instrument 602) and/or a force applied to the cannula or GW based at least on endo-therapeutic device information 633. Such device information may include specifications of an endo-therapeutic device such as inner and outer diameters, tip shape, tip load and/or stiffness, torquability (an ability of rotating element to overcome tuning resistance), bending angle, wire support (a measure or wire's resistance to a bending force), among others. An ML model may be trained to establish between the specifications of a cannula or GW and a proper insertion angle or force applied thereto. The cannulation and navigation parameter estimation unit 616 can feed the specifications of the cannula or GW presently used in the procedure to the trained ML model to estimate a proper insertion angle and/or force applied to the cannula or GW.

In an example, after the cannula/GW is inserted into duodenal papilla, the cannulation and navigation parameter estimation unit 616 can determine a direction, or a navigation path, for advancing the cannula/GW within the common bile duct or other portions of pancreaticobiliary system. Such direction or navigation path can be determined based on endoscopic images 631 and/or external image sources 632, such as images of the common bile duct or other portions of pancreaticobiliary system. The images can include one or more of endoscopic images obtained prior to or during an ERCP procedure, MRI images obtained prior to or during an MRCP procedure, among others. The cannulation and navigation parameter estimation unit 616 can determine the direction using a trained ML model 612. In an example, the ML model 612 can be trained using reinforcement learning. Reinforcement learning is a machine learning approach for creating behavior policies (e.g., the cannula/GW's heading direction) under certain states in an environment in order to maximize cumulative rewards associated with the behavior policies. In contrast to supervised learning which uses labelled input/output pairs to train the model, reinforcement learning maintains a balance between exploration of uncharted territory (e.g., different heading directions or paths to take within the common bile duct or the pancreaticobiliary system) and exploitation of current knowledge during the model training process. For example, reinforcement learning allows the model being trained to actively gather experience in situations where it performs poorly without needing external interventions, and can directly optimize behavior performance through the reward function. In the above example of ML- based determination of a heading direction or navigation path for the cannula/GW, the reinforcement learning can be advantageous especially with the lack of labelled training data such as from past procedures performed by a plurality of physicians on a plurality of patients. The navigation controller 620 can control the actuator 603 (or the robot arm) to orient the distal portion of the cannula/GW in accordance with the determined direction and navigate through the pancreaticobiliary system in accordance with the determined navigation path.

In some examples, the cannulation and navigation parameter estimation unit 616 can determine an insertion angle of a cannula/GW (an example of the steerable elongate instrument 602) based on past endoscopic procedure data stored in the endoscopic procedure database 606. The stored procedure data can include, for each procedure, endoscopic images or videos showing patient anatomy, cannulation and endoscope navigation routes, progress of cannulation and navigation, the physician's information, among other information. In an example, the stored procedure data can include, for each procedure, one or more cannulation or navigation parameters that are recorded during the procedure, or obtained by offline analysis the endoscopic images or videos. The stored procedure data may also include indications of physicians' habits or preferred procedure approaches. In various embodiments, at least a portion of the stored procedure data is used as a training set for training a ML model that predicts the cannulation and navigation parameter(s) of a procedure to be performed at a later time. In an example, the training data can be screened such that only data of procedures performed by certain physicians (such as those with substantially similar experience levels to the operating physician), and/or data of procedures on certain patients with special requirement (such as those with substantially similar anatomy or patient medical information to the present patient) are included in the training dataset. Additionally, in some examples, the endo-therapeutic device information 633, such as endoscopes used in ERCP procedures, may be included in the ML model training process. The cannulation and navigation parameter estimation unit 616 can determine, for example, the insertion angle of a cannula/GW using the trained ML model. The navigation controller 620 can then control the actuator 603 (or the robot arm) to position the distal portion of the cannula/GW and insert into the duodenal papilla in accordance with the determined insertion angle.

In some examples, an ML model may be trained using past imaging data and procedure data stored in the endoscopic procedure database 606 to produce multiple reference control patterns, such as multiple reference heading directions or reference navigation paths, multiple insertion angles, or multiple force levels or force ranges to apply to the steerable elongate instrument 602 (e.g., a cannula/GW). The multiple reference control patterns can be sorted, or otherwise categorized into groups, based on one or more of success rate, patient outcome and prognosis, procedure time, among other qualifications. The cannulation and navigation parameter estimation unit 616 can, based on the imaging data of the current patient, select from the reference control patterns at least one that corresponds to, for example, the highest success rate or a specified success rate (e.g., having a success rate exceeding 90%). The navigation controller 620 can then control the actuator 603 (or the robot arm) to control the positioning and motion of the cannula/GW in accordance with the selected reference control pattern. In addition, based on the feedback received from the live procedure, the cannulation and navigation parameter estimation unit 616 can switch from one reference control pattern to a different reference control pattern.

According to some examples, two or more different ML models can be separately trained, validated, and used in different applications including, for example, target anatomy recognition, cannulation or navigation parameter estimation, automatic endoscope positioning and navigation, automatic insertion of cannula or a guidewire (hereinafter a "cannula/GW") in the target anatomy, among others. For example, a ERCP procedure may involve multiple steps including passing an endoscope down to the duodenum and recognizing duodenal papilla, inserting a cannula/GW to the papilla, and adjusting direction or force of the cannula/GW to avoid excess pressure to the pancreas. Different ML models may be trained and used in respective steps of the ERCP procedure. In an example, to identify the location of papilla and locating the endoscope to be in front of papilla, a reinforcement learning model may be trained using endoscopic images 631 of papilla and endoscope control log data 636 to determine precise location of the endoscope relative to the papilla. The cannulation and navigation parameter estimation unit 616 can use said reinforcement learning model to determine if the endoscope is in front of papilla. The navigation controller 620 can generate a control signal to guide endoscope positioning if it is determined that the endoscope is not in front of papilla.

In another example, separated ML models may be trained to perform different functions, such as a first ML model being trained to recognize a region of interest, and a second ML model being trained to determine optimal cannulations parameters associated with the recognized region of interest. For example, to control the endoscope such that the papilla is captured in the center of the endoscope image and to guide insertion of a cannula/GW to the center of papilla from an optimal direction or position, a first supervised learning model may be trained to recognize duodenal papilla with certain spatial or geometrical characteristics thereof, such as the center of the papilla for endoscopic access or cannulation. Additionally, a second reinforcement learning model may be trained to determine an optimal direction to move the endoscope to capture the center of papilla. The first supervised learning model and the second reinforcement learning model can be separately trained each using one or more of endoscopic images 631 of papilla, external image sources 632 such as MRI image of bile duct, and endoscope control log data 636.

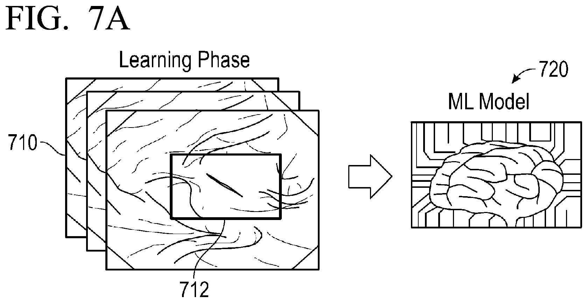

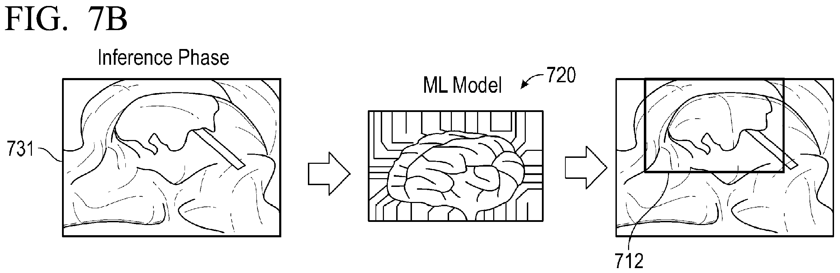

The target anatomy recognition unit 614 can use the trained supervised learning model to localize the center of duodenal papilla. FIGS. 7A-7B are diagrams illustrating an example of training a ML-based papilla recognition model and using the trained ML model to detect and localize papilla from a live endoscopic image. FIG. 7A illustrates an ML model training (or learning) phase during which a ML model 720 (e.g., a supervised learning model) may be trained using a plurality of endoscopic images 710 to determine, from an endoscopic image, a bordered zone of a particular shape, such as a rectangle 712, that encloses the papilla center with a high probability. FIG. 7B illustrates an inference phase during which the trained ML model 720 is applied to a live endoscopic image 731 to detect and localize a papilla center from the live endoscopic image. In an example, the target anatomy recognition unit 614 can superimpose the bordered zone, such as the rectangle 712, over the live endoscopic image 731, and detect the center of duodenal papilla within the bordered zone. The cannulation and navigation parameter estimation unit 616 can use the trained reinforcement learning model to determine if the endoscope is in an optimal direction to capture the center of papilla. The navigation controller 620 can generate a control signal to guide endoscope positioning if it is determined that the endoscope is not in an optimal position to capture the center of papilla.

In yet another example, after passing a cannula/GW through duodenal papilla and into the common bile duct, to adjust direction or force applied to the cannula/GW to avoid excessive pressure exerted to the pancreas and potential tissue damage, a first supervised learning model may be trained to estimate the force or load on the cannula/GW. Additionally, a second reinforcement learning model may be trained to determine an optimal insertion angle and force for inserting the cannula/GW within the common bile duct. The first supervised learning model and the second reinforcement learning model can be separately trained each using one or more of endoscopic images 631 of papilla, external image sources 632 such as MRI image of the area neighboring the papilla, sensor signals 634 such as force sensed by a sensor disposed at the distal portion of the cannula/GW (e.g., a strain gauge, a piezoelectric sensor, an inductive sensor, or a capacitive sensor), and endoscope control log data 636 (e.g., time-series changes in the movement and deflection of cannula/GW on the endoscopic image). The cannulation and navigation parameter estimation unit 616 can use the trained supervised learning model to estimate the force or load on the cannula/GW, and use the trained reinforcement learning model to estimate insertion angle and force applied to the cannula/GW. The navigation controller 620 can generate a control signal to guide cannula/GW positioning.

The contrast and scan parameter estimation unit 618 can automatically estimate one or more contrast and scan parameters using a trained ML model, such as the trained ML model 612 or a portion thereof. By way of example and not limitation, the contrast and scan parameters may include volume (dose) of contrast medium to be administered, flow rate of the contrast medium, injection duration, and an injection site for administering the contrast medium (e.g., through the papilla), among other parameters. In an example where the contrast medium is administered via a delivery tool such as an endoscope or other injection devices, the contrast and scan parameters may include position, direction, orientation, or posture of the injection device or the endoscope. The contrast and scan parameters may additionally or alternatively include scan timing (i.e., timing to take images of the anatomical target or other regions of interest), scan duration (radiation dose), scan direction, contrast bolus arrival time, or a scan delay from the contrast medium injection, among other parameters. For example, to obtain high-quality images, the imaging scan timing may be set at or around the peak contrast enhancement in the targeted region of interest.

The ML model, as discussed above, can be trained using supervised learning, deep learning, unsupervised learning, or reinforcement leaning, among other algorithms or methodologies. To train an ML-based contrast and scan parameter model, a training dataset can be constructed using past patient data from the input data sources 630, including, for example, endoscopic images 631, external image sources 632 during past imaging examination (e.g., X-rays, fluoroscopy, CT, or MRI scan) and patient information 635, collected from a plurality patients. The training dataset may also include, for each endoscopic image in the training dataset, contrast and scan parameters used to produce the radiologic images (e.g., X-rays, fluoroscopy, CT, or MRI images) at the time of taking the endoscopic image. In some examples, patient information (e.g., morphological characteristics such as presence of stenosis or stricture in target anatomy), device information (e.g., specification of endoscopes or contrast medium injection devices such as size, shape, distal end curvatures), and contrast medium characteristics (e.g., viscosity of contrast medium) may also be included in the training dataset. As discussed above, in some examples, the training data can be screened based on physicians performing the procedure of the same or similar type, patients with substantially similar anatomy or medical information to the present patient, success rate of the procedure, or complication associated with the patients, among other considerations. The training of the ML model involves algorithmically adjusting one or more ML model parameters until the ML model being trained satisfies a specified training convergence criterion.

The trained ML model may establish a correspondence between the endoscopic images (optionally along with other information such as external images, patient information, device information, contrast medium characteristics) and optimal or desired contrast and scan parameters. The trained ML model can be used by the contrast and scan parameter estimation unit 618 to estimate, for a given image of a target anatomy (e.g., a real-time endoscopic image obtained during an endoscopic procedure such as ERCP) and a particular endoscope or injection device, one or more of optimal contrast and scan parameters, such as an optimal dose of contrast medium, an optimal location for administering the contrast medium, optimal position and orientation of the injection device or the endoscope for injecting the contrast medium, or an optimal timing to take an imaging scan.

The navigation controller 620 can control the actuator 603 (such as a robot arm) to robotically facilitation operation of an endoscope (an example of the steerable elongate instrument 602), or an injection device, to deliver the contrast medium of an optimal dose, at an optimal location, and in an optimal manner in accordance with the determined contrast and scan parameters. Additionally or alternatively, the navigation controller 620 can provide a control signal to an imaging device or system (e.g., an endoscopic imaging device, an X-ray or fluoroscopy system) to time the imaging device or system to taken images, or to determine a scan duration, scan direction, or scan delay with respect to contrast injection in accordance with the determined contrast and scan parameters.

The ML model based estimation or contrast and scan parameters with respect to contrast medium administration and imaging/scan timing as described herein can improve the efficiency of contrast and radiation management, reduce radiation exposure while maintaining image quality, thereby enhancing patient safety and diagnostic efficacy.

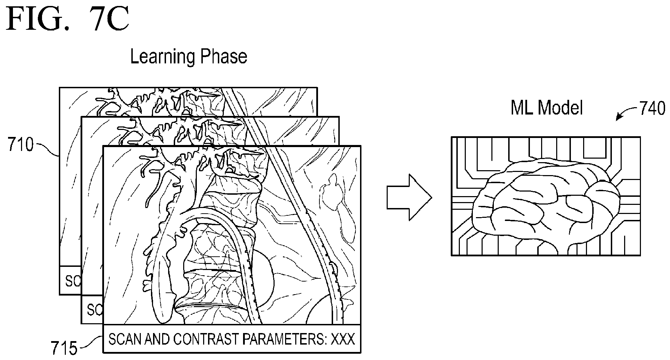

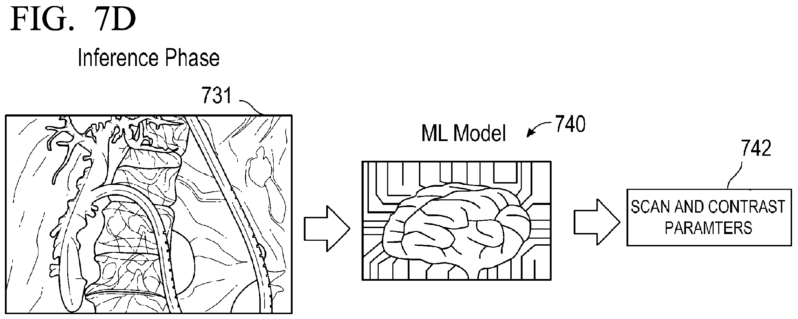

FIGS. 7C-7D are diagrams illustrating an example of training a ML model, and using the trained ML model to estimate one or more contrast and scan parameters for a given endoscopic image of the target area. The estimated contrast and scan parameters may be used to guide imaging scan during an endoscopic procedure. FIG. 7C illustrates an ML model training (or learning) phase during which a ML model 740 (e.g., a supervised learning model) may be trained using a plurality of endoscopic images 710 and contrast and scan parameters 715 associated with each image. FIG. 7D illustrates an inference phase during which the trained ML model 740 is applied to a live endoscopic image 731 to estimate one or more "optimal" or desired contrast and scan parameters 742, such as by using the contrast and scan parameter estimation unit 618. The navigation controller 620 can robotically facilitate operation of an endoscope or an injection device to administer the contrast medium in accordance with the "optimal" or desired contrast and scan parameters 742. Additionally or alternatively, the navigation controller 620 can provide a control signal to an imaging device or system to adjust imaging of the target anatomy in accordance with the estimated one or more scan parameters.

The user interface device 640 can include an output unit 642 and an input unit 645, which are examples of the output unit 18 and the input unit 20 respectively as shown in FIG. 2. The output unit 642 can include a display 643 that can display images of the target anatomy as recognized by the target anatomy recognition unit 614. In some examples, the display 643 can present a visual presentation of the cannulation and navigation parameters produced by the cannulation and navigation parameter estimation unit 610. In an example, the displayed region of the target anatomy images can be automatically adjusted according to the position or direction of a distal end of the endoscope relative to an anatomical target. For example, the output unit 642 may automatically zoom in an image as the endoscope tip gets closer to the papilla to show more details of the papilla. Alternatively, the zooming function can be activated and adjusted manually by the user (e.g., operating physician) via the input unit 645. In an example, the output unit 642 can display a cross-section view of an anatomy in a direction specified by a user, such as via the input unit 645. In an example, the user may adjust viewing angle (e.g., rotating the view) and have a 360-degree view of the reconstructed or integrated 3D images via the input unit 645. In an example, at least a portion of the input unit 645 can be incorporated into the endoscope, such as the handle section 32 of endoscope 14, to facilitate user operation during the procedure.

In some examples, the display 643 may automatically center the target anatomy in a viewing area, such as based on the distance and viewing angle of the imaging device (e.g., camera) relative to the target anatomy. In an example, the controller 601 can control the positioning and direction of the endoscope to adjust viewing angle of the imaging device to achieve auto-centering of the target anatomy. Additionally or alternatively, the controller 601 can control an image processor to post-process the acquired image including re-positioning the identified target anatomy at the center of the viewing area.

In some examples, the output unit 642 may display on the image a visual indication of the target anatomy (e.g., duodenal papilla), a projected navigation path toward the target anatomy; or a progression of the endoscope toward the targe anatomy along the projected navigation path. Display settings can be adjusted by the user via the input unit 645. The visual indication may take the format of markers, annotations (icons, texts, or graphs), highlights, or animation, among other visual indicators. For example, markers of different shapes, colors, forms, or sizes can be display on the reconstructed or integrated image to distinguish different tissue, anatomical regions, their accessibility or criticality.

In some examples, the output unit 642 may display the estimated contrast and scan parameters generated by the contrast and scan parameter estimation unit 618 to a user (e.g., an endoscopist). The user may use the estimated contrast and scan parameters as a guide to assist in administering the contrast medium or taking imaging scans of the target anatomy during an endoscopic procedure.

The output unit 642 can include an alert and feedback generator 644 that can generate an alert, a notification, or other formats of human-perceptible feedback to the operating physician on the status or progress of the cannulation or navigation in reference to the navigation plan. For example, an alert can be generated to indicate a risk of tissue damage associated with improper cannulation. The feedback can be in one or more forms of audio feedback, visual feedback, or haptic feedback. For example, when the endoscope tip enters or comes closer to a "critical zone" (e.g., proximity sensor detecting a distance to a critical anatomy of interest shorter than a threshold distance), the critical zone can be shown in different colors to represent such distance (e.g., green zone, yellow zone, and read zone as the endoscope gets closer to the critical zone). Additionally or alternatively, haptic feedback such as touch or vibration may be generated and felt by the operating physician. In an example, the alert and feedback generator 644 can automatically adjust the vibration strength according to the distance to the critical zone. For example, a low vibration can be generated when the endoscope tip is in a green zone. If the system predicts, based on present advancing speed and direction of the endoscope, that the endoscope tip will reach the critical zone in a time lower than a predetermined threshold, then alert and feedback generator 644 can apply moderate vibration when the endoscope tip reaches a yellow zone, and apply high vibration when the endoscope tip reaches red zones to indicate a risk of tissue damage. The real-time alert and feedback in an image-guided endoscopic procedure as described herein can improve the efficiency of cannulation and endoscope navigation, especially for inexperienced physicians, and can improve endoscopic procedure success rate and patient outcome.

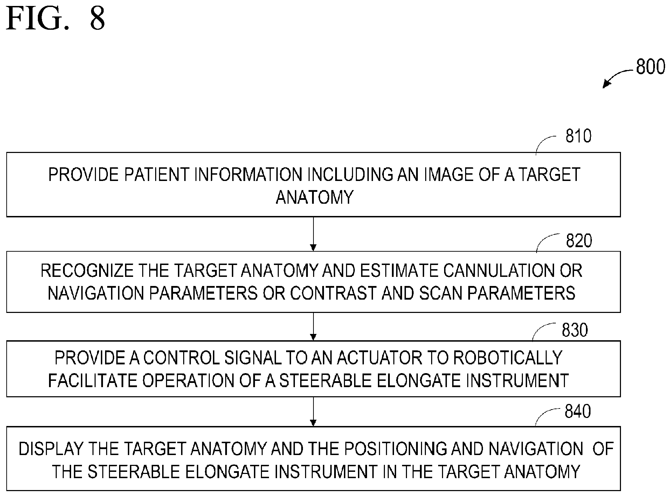

FIG. 8 is a flow chart illustrating an example method 800 for robotically positioning and navigating a steerable elongate instrument in a target anatomy via an endoscopic system. The steerable endoluminal instrument can include diagnostic or therapeutic endoscopes, cannulas, catheters, guidewires, or guide sheaths, among others. The method 800 may be implemented in and executed by the robotic endoscopic surgical system 500. Although the processes of the method 800 are drawn in one flow chart, they are not required to be performed in a particular order. In various examples, some of the processes can be performed in a different order than that illustrated herein.

At 810, patient information including an image of the target anatomy can be provided for use in a robotically assisted endoscopic procedure. The image of the target anatomy may include endoscopic images of duodenal papilla and its surrounding environment captured by an imaging sensor on the endoscope during an endoscopic procedure, or images from other sources including, for example, X-ray or fluoroscopy images, electrical potential map or an electrical impedance map, CT images, MRI images such as images obtained from Magnetic resonance cholangiopancreatography (MRCP) procedures, or acoustic images such as endoscopic ultrasonography (EUS) images, among others. In addition to the images of the target anatomy, other information may be used in a robotically assisted endoscopic procedure, which may include endo-therapeutic device information, sensor signals, physician information (e.g., the operating physician's habits or preference of using the steerable elongate instrument), and endoscope control log data, as described above with reference to FIG. 6.

At 820, the images of the target anatomy, and optionally other information received at step 810, may be provided to a trained machine-learning (ML) model to recognize the target anatomy, and estimate one or more cannulation or navigation parameters for maneuvering the steerable elongate instrument and/or one or more contrast and scan parameters for an imaging scan of the target anatomy. Examples of the cannulation or navigation parameters may include: a position of the distal portion (e.g., the functional section 30 of the endoscope 14 as shown in FIG. 1) of an endoscope or other steerable elongate instrument relative to an anatomical target of interest, such as a distance from the endoscope distal portion to duodenal papilla; a heading direction of the distal portion of the steerable elongate instrument relative to the anatomical target; an insertion angle of a cannula or a surgical element used in cannulation; a protrusion amount of a cannula or a surgical element; a speed or a force applied to the endoscope distal portion or a surgical element; a rotational direction or a cutting area of a surgical element; and a projected navigation path toward the anatomical target of interest. Examples of the contrast and scan parameters may include volume (dose) of contrast medium; flow rate of the contrast medium, injection duration; an injection site for administering the contrast medium (e.g., through the papilla); position, direction, orientation, or posture of the injection device or the endoscope; scan timing (i.e., timing to take images of the anatomical target or other regions of interest); scan duration (radiation dose); scan direction; contrast bolus arrival time; or a scan delay from the contrast medium injection, among other parameters.

The ML model may be trained using supervised learning, unsupervised learning, or reinforcement leaning. The ML model may be trained using past procedure data stored in an endoscopic procedure database 606. The past procedure data stored in a database that stores procedure data acquired during respective endoscopic procedures performed by a plurality of physicians on a plurality of patients.

At 830, a control signal may be provided to an actuator (as a motor actuating a robot arm) to robotically facilitate operation of the steerable elongate instrument (e.g., adjusting the position or navigation) in the target anatomy in accordance with the one or more cannulation or navigation parameters determined at step 820. In an example, a control signal may be provided to an actuator to robotically assist operation of an endoscope or an injection device to deliver the contrast medium of an optimal dose, at an optimal location, and in an optimal manner in accordance with the determined contrast and scan parameters. In an example, a control signal may be provided to an actuator to robotically assist in an operation of an imaging device or system in timing the imaging scan, or determining scan duration, scan direction, or scan delay with respect to contrast injection in accordance with the determined contrast and scan parameters.

Various examples of cannulation or navigation parameter estimation and robotically assisted positioning and navigation are discussed in this document. In one example, a trained ML model may be used to estimate a force applied to a cannula or GW (an example of the steerable elongate instrument) based on a distance from the distal tip of the cannula/GW to duodenal papilla. The distance may be measured using a proximity sensor, or estimated from an endoscopic image. The force applied to the cannula or GW can be inversely proportional to said distance. Dynamically adjusting the force based on the distance to the critical anatomy (e.g., duodenal papilla, common bile duct, and pancreas) as described herein can avoid or reduce damage to pancreatic parenchyma caused by cannula/GW. In another example, an ML model may be trained using reinforcement learning, and used to determine a direction, or a navigation path, for advancing the cannula/GW within the common bile duct or other portions of the pancreaticobiliary system. In yet another example, an ML model may be trained to estimate an insertion angle of a cannula/GW based on past endoscopic procedure data stored in the endoscopic procedure database.

In an example, an ML model may be trained using reinforcement learning to determine precise location of the endoscope relative to the papilla, and determine if the endoscope is in front of papilla. If the endoscope is not in front of the papilla, a control signal can be provided to an actuator to automatically adjust endoscope position.

In another example, to control the endoscope such that the papilla is captured in the center of the endoscope image and to guide insertion of a cannula/GW to the center of papilla from an optimal direction or position, a first ML model may be trained using supervised learning to recognize duodenal papilla with certain spatial or geometrical characteristics thereof, such as the center of the papilla for endoscopic access or cannulation. A second ML model may be trained using reinforcement learning to determine an optimal direction to move the endoscope to capture the center of papilla.

In yet another example, after passing a cannula/GW through duodenal papilla and into the common bile duct, to adjust direction or force applied to the cannula/GW to avoid excessive pressure exerted to the pancreas and potential tissue damage, a first ML model may be trained using supervised learning to estimate the force or load on the cannula/GW. Additionally, a second ML model may be trained using reinforcement learning to determine an optimal insertion angle and force for inserting the cannula/GW within the common bile duct. A control signal can be provided to the actuator to robotically control cannula/GW positioning.

At 840, the target anatomy and the process of positioning and navigation of the steerable elongate instrument in the target anatomy, may be displayed on an output unit. A visual presentation of the cannulation and navigation parameters may also be displayed. In an example, the displayed region of the target anatomy images can be automatically adjusted according to the position or direction of a distal end of the endoscope relative to an anatomical target. Such automatic adjustment may include auto-zooming the target anatomy, auto-centering the target anatomy in a viewing area, and automatic adjustment of viewing angles. In an example, visual indication of one or more of an anatomical target, a projected navigation path toward the anatomical target, or a progress of the endoscope advancing toward the anatomical target along the projected navigation path may be displayed overlaid upon the image being displayed on the output unit. In some examples, the estimated contrast and scan parameters may be displayed to a user and assist in administering the contrast medium or taking imaging scans of the target anatomy during an endoscopic procedure. In some examples, an alert, a notification, or other formats of human-perceptible feedback may be provided to the operating physician to indicate the status or progress of the cannulation or navigation.

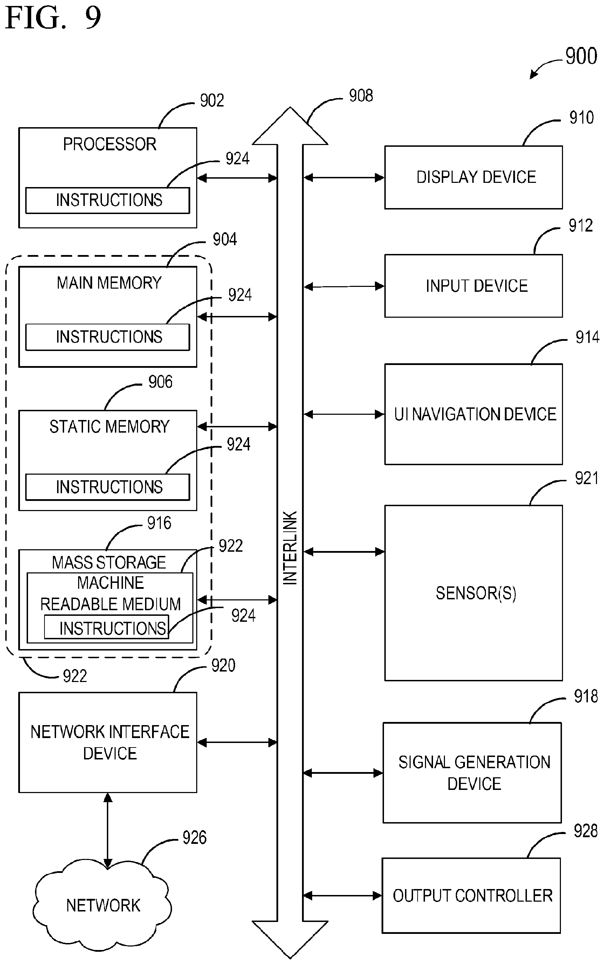

FIG. 9 illustrates generally a block diagram of an example machine 900 upon which any one or more of the techniques (e.g., methodologies) discussed herein may perform. Portions of this description may apply to the computing framework of various portions of the robotically assisted navigation system 600, such as the navigation planning unit 610.

In alternative embodiments, the machine 900 may operate as a standalone device or may be connected (e.g., networked) to other machines. In a networked deployment, the machine 900 may operate in the capacity of a server machine, a client machine, or both in server-client network environments. In an example, the machine 900 may act as a peer machine in peer-to-peer (P2P) (or other distributed) network environment. The machine 900 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 900 may include a hardware processor 902 (e.g., a central processing unit (CPU), a graphics processing unit (GPU), a hardware processor core, or any combination thereof), a main memory 904 and a static memory 906, some or all of which may communicate with each other via an interlink (e.g., bus) 908. The machine 900 may further include a display unit 910 (e.g., a raster display, vector display, holographic display, etc.), an alphanumeric input device 912 (e.g., a keyboard), and a user interface (UI) navigation device 914 (e.g., a mouse). In an example, the display unit 910, input device 912 and UI navigation device 914 may be a touch screen display. The machine 900 may additionally include a storage device (e.g., drive unit) 916, a signal generation device 918 (e.g., a speaker), a network interface device 920, and one or more sensors 921, such as a global positioning system (GPS) sensor, compass, accelerometer, or other sensors. The machine 900 may include an output controller 928, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 916 may include a machine readable medium 922 on which is stored one or more sets of data structures or instructions 924 (e.g., software) embodying or utilized by any one or more of the techniques or functions described herein. The instructions 924 may also reside, completely or at least partially, within the main memory 904, within static memory 906, or within the hardware processor 902 during execution thereof by the machine 900. In an example, one or any combination of the hardware processor 902, the main memory 904, the static memory 906, or the storage device 916 may constitute machine readable media.

While the machine-readable medium 922 is illustrated as a single medium, the term "machine readable medium" may include a single medium or multiple media (e.g., a centralized or distributed database, and/or associated caches and servers) configured to store the one or more instructions 924.

The term "machine readable medium" may include any medium that is capable of storing, encoding, or carrying instructions for execution by the machine 900 and that cause the machine 900 to perform any one or more of the techniques of the present disclosure, or that is capable of storing, encoding or carrying data structures used by or associated with such instructions. Non-limiting machine-readable medium examples may include solid-state memories, and optical and magnetic media. In an example, a massed machine-readable medium comprises a machine readable medium with a plurality of particles having invariant (e.g., rest) mass. Accordingly, massed machine-readable media are not transitory propagating signals. Specific examples of massed machine-readable media may include: non- volatile memory, such as semiconductor memory devices (e.g., Electrically Programmable Read-Only Memory (EPROM), Electrically Erasable Programmable Read-Only Memory (EPSOM)) and flash memory devices; magnetic disks, such as internal hard disks and removable disks; magneto-optical disks; and CD-ROM and DVD-ROM disks.

The instructions 924 may further be transmitted or received over a communication network 926 using a transmission medium via the network interface device 920 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, among others. In an example, the network interface device 920 may include one or more physical jacks (e.g., Ethernet, coaxial, or phone jacks) or one or more antennas to connect to the communication network 926. In an example, the network interface device 920 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 900, and includes digital or analog communications signals or other intangible medium to facilitate communication of such software.

### (Additional Notes)

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described.

However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. An endoscopic system, comprising:
a steerable elongate instrument configured to be positioned and navigated in a target anatomy of a patient; and
a controller circuit configured to:
receive patient information including an image of the target anatomy;
apply the image of the target anatomy to at least one trained machine-learning (ML) model to recognize the target anatomy and to estimate one or more contrast and scan parameters for an imaging scan of the target anatomy; and
provide the estimated one or more contrast and scan parameters to a user or a process.

2. The endoscopic system of claim 1, wherein the received image of the target anatomy include an endoscopic image.

3. The endoscopic system of any of claims 1-2, wherein the at least one trained ML model includes a first trained ML model and a second trained ML model different from the first trained ML model, and
wherein the controller circuit is configured to apply the image of the target anatomy to the first trained ML model to recognize the target anatomy, and to apply the image of the target anatomy to the second trained ML model to estimate the one or more contrast and scan parameters for the imaging scan of the target anatomy.

4. The endoscopic system of any of claims 1-3, wherein the controller circuit is further configured to provide a control signal to an actuator to robotically facilitate (i) administration of contrast medium via the steerable elongate instrument or (ii) imaging of the target anatomy in accordance with the estimated one or more contrast and scan parameters.

5. The endoscopic system of claim 4, wherein:
the steerable elongate instrument includes an endoscope or a steerable injection device, and the estimated one or more contrast and scan parameters include a contrast medium parameter; and
the controller circuit is configured to provide the control signal to the actuator to robotically facilitate operation of the steerable elongate instrument to administer the contrast medium in accordance with the contrast medium parameter.

6. The endoscopic system of claim 5, wherein the contrast medium parameter includes one or more of a volume, a flow rate, or an injection duration of the contrast medium.

7. The endoscopic system of any of claims 5-6, wherein the target anatomy includes a portion of a pancreaticobiliary system of the patient, and the contrast medium parameter includes a contrast medium injection site of the pancreaticobiliary system.

8. The endoscopic system of claim 4, wherein:
the estimated one or more contrast and scan parameters include a scan parameter for imaging the target anatomy; and
the controller circuit is configured to provide the control signal to the actuator to robotically facilitate operation of an imaging device to take an imaging scan of the target anatomy in accordance with the scan parameter.

9. The endoscopic system of claim 8, wherein the scan parameter includes one or more of a scan timing, scan duration, scan direction, or a scan delay with respect to the administration of the contrast medium.

10. The endoscopic system of any of claims 1-9, wherein:
the target anatomy includes a portion of a pancreaticobiliary system of the patient; and
the controller circuit is configured to apply the image of the target anatomy to the trained ML model to recognize stenosis in the pancreaticobiliary system.

11. The endoscopic system of any of claims 1-10, comprising a training module configured to train the ML model using a training dataset comprising procedure data from past endoscopic procedures on a plurality of patients, the procedure data comprising (i) endoscopic images of target anatomies of the plurality of patients, and (ii) assessments of respective contrast and scan parameters used to produce the endoscopic images.

12. The endoscopic system of claim 11, wherein the training module is configured to train the ML model using supervised learning or deep learning.

13. The endoscopic system of any of claims 1-12, wherein the controller circuit is configured to estimate the one or more contrast and scan parameters further using viscosity information of a contrast medium.

14. The endoscopic system of any of claims 1-13, comprising an output unit configured to present the estimated one or more contrast and scan parameters to a user to assist the user in administering a contrast medium or imaging the target anatomy.

15. A method of robotically maneuvering an steerable elongate instrument in a target anatomy and controlling imaging scan of the target anatomy via an endoscopic system, the method comprising:
providing patient information including an image of the target anatomy;
applying the target anatomy image to at least one trained machine-learning (ML) model and recognizing the target anatomy and estimating one or more contrast and scan parameters for an imaging scan of the target anatomy; and
providing the estimated one or more contrast and scan parameters to a user or a process.

16. The method of claim 15, wherein applying the target anatomy image to at least one trained ML model includes:
applying the image of the target anatomy to a first trained ML model to recognize the target anatomy; and
applying the image of the target anatomy to a second trained ML model to estimate the one or more contrast and scan parameters for the imaging scan of the target anatomy, the second trained ML model different from the first trained ML model.

17. The method of any of claims 15-16, further comprising providing a control signal to an actuator to robotically facilitate (i) administration of contrast medium via the steerable elongate instrument or (ii) imaging of the target anatomy in accordance with the estimated one or more contrast and scan parameters.

18. The method of claim 17, wherein:
the estimated one or more contrast and scan parameters include a contrast medium parameter for administering a contrast medium; and
the control signal is provided to the actuator to robotically facilitate operation of the steerable elongate instrument to administer the contrast medium is in accordance with the contrast medium parameter.

19. The method of claim 18, wherein the contrast medium parameter includes one or more of a volume, a flow rate, or an injection duration of the contrast medium.

20. The method of any of claims 18-19, wherein the target anatomy includes a portion of a pancreaticobiliary system of the patient, and the contrast medium parameter includes a contrast medium injection site of the pancreaticobiliary system.

21. The method of claim 17, wherein:
the estimated one or more contrast and scan parameters include a scan parameter for imaging the target anatomy; and
the control signal is provided to the actuator to robotically facilitate operation of an imaging device to take an imaging scan of the target anatomy in accordance with the scan parameter.

22. The method of claim 21, wherein the scan parameter includes one or more of a scan timing, scan duration, scan direction, or a scan delay with respect to the administration of the contrast medium.

23. The method of any of claims 15-22, comprising training the ML model, via a training module of the endoscopic system, using a training dataset comprising stored endoscopic procedure data of a plurality of patients, the stored data comprising (i) endoscopic images of target anatomies of the plurality of patients, and (ii) assessments of respective contrast and scan parameters used to produce the endoscopic images.

24. The method of any of claims 15-23, comprising presenting the estimated one or more contrast and scan parameters to a user via an output unit to assist the user in administering a contrast medium or imaging the target anatomy.
